# EUROPEAN PATENT APPLICATION

(11) **EP 0 572 364 A2**
(43) Date of publication of application: **01.12.1993**
(21) Application number: 93830229.6
(22) Date of filing: 26.05.1993
(51) Int. Cl.: C12N 5/08, A61F 9/00, A61L 27/00

(54) **Differentiated ocular surface epithelial cell cultures, process for the preparation and carrier for uses thereof**

(30) Priority: 29.05.1992 IT RM920408
(71) Applicant: ISTITUTO NAZIONALE PER LA RICERCA SUL CANCRO, I-16132 Genova (IT); CELLIFE BIOTECNOLOGIE PER LA VITA S.r.l., I-20126 Milano (IT)
(72) Inventor: Cancedda, Ranieri, c/o Istituto Nazionale Per La, I-16132 Genova (IT); De Luca, Michele, c/o Istituto Nazionale Per La, I-16132 Genova (IT); Gherzi, Roberto, c/o Istituto Nazionale Per La, I-16132 Genova (IT)
(74) Representative: de Simone, Domenico

(57) **Abstract**

A process to cultivate *in vitro* diffrentiated ocular surface epithelial cells. Said cells are useful to eye transplantation by means of suitable carriers.

## Description

This invention relates to differentiated ocular surface epithelial cells, generated by *in vitro* cultivation of eye biopsies, to the process for the production and to carriers for the use thereof.

The anterior ocular surface is covered with two types of genotypically different and highly specialized epithelia, namely the conjunctival and the limbal/corneal epithelium. The bulbar conjunctival epithelium consists of six to nine loosely organized cell layers overlying a thin basement membrane, it is well vascularized and it is populated by mucin secreting goblet cells (Friend J, Kenyon KR. In: Smolin G, Thoft RA (eds): The cornea. Scientific foundationand clinical practice. Boston: Little, Brown and Co, 1987;16.Thoft RA, Friend J. Invest Ophthalmol Visual Sci 1977;16:14). The corneal epithelium is a four to five layered stratified squamous epithelium with a cuboid basal layer, it is extremely flat with no papillary structures, it is devoided of goblet cells as well as other cell types, and it lyes on the avascular corneal stroma via the Bowman's layer (ibid.); the corneal stem cell resides in the limbus which is the narrow transitional zone of well vascularized epithelium between the cornea and the bulbar conjunctiva. The limbus consists of up to 12 layers of epithelial cells organized in well developed rete ridges, it lacks goblet cells but it is populated by Langerhans cells and melanocytes (ibid.). Limbal cells are the progenitors of corneal, but not conjunctival cells (Shermer A, Galvin S, Sun TT. J Cell Biol 1986;103: 49. Cotsarelis G, Cheng SZ, Dong G, Sun TT, Lavker RM. Cell 1989; 57: 201.Tsai RJF, Sun TT, Tseng SCG. Ophthalmology 1990; 97: 446. Dua H, Forrester JV. Am J Ophthalmol. 1990; 110: 646).

Visual acuity is strictly dependent upon corneal epithelium, whose integrity is maintained through the circadian, centripetal migration of corneal stem cells (and in turn of rapidly dividing "transient amplyfing" cells from the eye limbus (Shermer A, et al; Cotsarelis G. et al.; Tsai RJFet al. ibid.). Extensive limbal/corneal epithelial destruction by chemical or thermal injuries, or by several other ocular pathologies, leads to corneal re-epithelialization by bulbar conjunctival cells (Tsai RJF et al. ibid.; Dua H, et al. ibid.; McCulley JP. Chemical injuries. In: Smolin G., Thoft RA. eds. The cornea: scientific foundations and clinical practice, 2nd ed. Boston: Little, Brown and Co. 1987; 527). This process is followed by neo-vascularization, chronic inflammation, recurrent epithelial defects and stromal scarring (ibid.). Therefore, it has been proposed that the autologous graft of conjunctival flaps from the fellow eye (Thoft RA. Conjunctival transplantation. Arch Ophthalmol. 1977; 95:1425-7) should include limbal epithelium (Tsai RJF et al. ibid.; Wei ZG,et al. ibid.; Kenyon KR, Tseng SCG. Ophthalmology. 1989; 96: 709). However, autologous limbal transplantation requires large limbal withdrawal from the fellow eye, and cannot be applied to wide and severe mono- or bi-lateral lesions. In these cases, corneal "conjunctivalization" often leads to a complete blindness which cannot be solved by traditional surgical techniques (Tsai RJF et al. ibid.; Dua H, et al. ibid.; McCulley JP. ibid.).

Traumas from alkaline agent burns, or patologies, such as the penphigoid cicatrix, the Stevens-Johnson syndrome, the ophtalmoxerosis and the "dry eye" syndrome, cause a loss of the muciferous cells or the physiological activity thereof, leading to a drastic mucin lowering, associated to an even full sight loss.

Some methods to cultivate *in vitro* epithelium cells (keratinocytes) of the skin are well known in the existing art (such as described in the US patents nr.4,304,866; 4,016,036; 4,456,687 and in the patent application WO 90/01266).

Attempts to cultivate *in vitro* conjunctival mucose cells, representing the whole of the *in vivo* tissue differentiated cells, particularly comprising the muciferous cells, did not succeed (Sun T-T and H. Green, "Nature", 269, 489-493, 1977).

The authors of the present invention have found that epithelial cells serially cultivated from a 1-2 mm² biopsy obtained from the human eye surface epithelium, reconstitute *in vitro* sheets of stratified ocular epithelium suitable for autologous graft. This technique may offer an alternative to patients suffering from bilateral extensive destruction of the limbal/corneal epithelium. In the case the biopsy is performed by either the eye limbus, or the corneal epithelium, then a sheet of corneal epithelium is preferentially obtained; in the case the biopsy is performed by either the fornix or the conjunctiva, a sheet of conjunctival mucosa is preferentially obtained.

The cells are able to express *in vitro* the differentiated phenotype of the cells of the donor site and are, therefor, suitable for the production of ocular epithelium-carrier complexes, to be used in transplantations.

Accordingly, an object of the invention is a process to grow *in vitro* differentiated ocular surface epithelial cells, comprising the following steps:
- drawing biopsies from a donor site of the ocular surface of the eye;
- treating said biopsies with cell dissociating agents to obtain dissociated cells;
- cultivating *in vitro* said cells on a solid carrier until a confluent layer of differentiated cells having at least one phenotypic feature of cells of said donor site is obtained;
- separating said layer from said solid carrier by means of a detaching agent.

In a preferred embodiment of the invention said donor site comprises eye limbus and/or perilimbus areas; alternatively said donor site comprises the eye fornix and/or conjunctiva.

Preferably said biopsies are freshly used, alternatively are frozen before using.

Preferably said treatment with cell dissociating agents comprises more than one incubation therewith, each one followed by a removal of the supernatant containing dissociated cells.

Preferably said dissociating agents comprise trypsin and bivalent ion chelating agents, more preferably EDTA, and most preferably said trypsin has a concentration in the range 0.01-0.1 % (w/v), and said EDTA has a concentration in the range 0.005-0.02 % (w/v).

Preferably, said *in vitro* culture is carried out in the presence of feeder layer of lethally irradiated cells and with a suitable culture medium for epithelial cells, preferably added with general or specific epithelium growth factors.

In a preferred embodiment of this invention, said culture comprises several cell passages in order to obtain no primary cultures, preferably two steps in order to obtain secondary cultures.

In an other preferred embodiment said cultures are frozen.

Another object of this invention is an *in vitro* culture of differentiated ocular surface epithelial cells from an ocular biopsy expressing at least one phenotypic feature of cells of the donor site, preferably obtained according to the process of the invention.

It is a further object of the invention an ocular epithelium-carrier complex to be used for eye transplantation, comprising a solid carrier and a culture of differentiated ocular surface epithelial cells according to this invention. Preferably said solid carrier comprises a sterile gauze, alternatively semirigid lens.

It is a further object of the invention a process for the preparation of an ocular epithelium-carrier complex to be used for eye transplantation, including semirigid lens and a culture of differentiated ocular surface epithelial cells, comprising the steps of:
- positioning said culture with the outer surface thereof upwards and leaning the convex surface of a first semirigid lens against it;
- turning the borders of said culture onto the concave surface of said first lens and fastening said borders by positioning the convex surface of a second lens on the concave surface of said first lens;
- turning the ocular epithelium-carrier complex in such a way, that the convex surfaces of said lens become concave and "viceversa", so that the epithelium basal layer, which is opposite to said outer surface, covers the concave surface of the first lens.

This invention shall be described in the following with reference to some explanatory, but not limiting examples.

### Example 1 Ocular surface epithelium culture in vitro

The whole process is carried out under sterile conditions.

Cells are cultivated from a 1-2 mm² biopsy taken from the eye limbus or perilimbus areas of cadavers or selected patients. Briefly, biopsies are minced and trypsinized (0.05% trypsin/0.01 %EDTA) at 37 °C for 3 h. Cells are collected every 30 min, plated (2.5 x 10⁴/cm²) on lethally irradiated 3T3-J2 cells (ATCC CRL 6473) (2.4 x 10⁴/cm²) and cultured in 5% CO₂ and humidified atmosphere in keratinocyte growth medium (KGM): Dulbecco-Vogt Eagle's (DMEM) and and Ham's F12 media (3:1 mixture) containing fetal bovine serum (10%), insulin (5 µg/ml), transferrin (5 µg/ml), adenine (0.18 mM), hydrocortisone (0.4 µg/ml), cholera toxin (0.1 nM), triiodothyronine (2 nM), epidermal growth factor (10 ng/ml), glutamine (4 mM), penicillin-streptomycin (50 IU/ml). Sub-confluent primary cultures are trypsinized, cells are plated in secondary cultures at a density of 4 x 10³ to 1.3 x 10⁴ cells/cm² and cultured as above. 3T3-J2 cells are cultured in DMEM containing bovine serum (10%), glutamine (4 mM) and penicillin-streptomycin (50 IU/ml). Confluent secondary cultures are detached from the surface of the vessel with the neutral protese Dispase 11(2.5 mg/ml in DMEM containing glutamine and penicillin-streptomycin, 45-60 min. at 37 °C) and grafted onto patients as described below.

In preliminary experiments, cultured epithelial sheets are transplanted onto nude mice. The obtained epithelium layers are placed on petrolatum gauzes (Adaptic from Johnson & Johnson) and then transplanted on athymic mouse "cutis vera", as described by Barrandon Y., Vincent Li B.S. and Green H.J. "Invest. Dermatol.", 91, 315, 1988.

### EXAMPLE 2 Transplantation

Preliminary experiments are performed on cells isolated and cultured from cadaver limbus. Colony forming efficiency and growth rate are greater than parallel cultures from bulbar conjunctiva. A stratified squamous epithelium resembling the corneal epithelium in its stratification pattern, absence of goblet cells and diffuse PAS staining is obtained from 16 out of 20 limbal biopsies. Shermer et al. (ibid.), describe the distribution of a basic keratin molecule (K3) which, within the anterior ocular epithelium, is a specific marker for corneal differentiation, and identified the limbal derived corneal stem cell on the basis of the expression pattern of this keratin. *In vitro* reconstituted epithelium stained positively for K3, further suggesting its corneal nature. Parallel staining of conjunctival epithelium, *in vitro* and *in vivo,* show undetectable levels of K3. The sum of these data leads to the conclusion that human limbal-derived epithelial cells reconstitute *in vitro* a true corneal epithelium.

In some delimited areas of epithelial sheets obtained from the remaining 4 limbal biopsies, the presence of cells identified as goblet cells on the basis of their shape and PAS staining is evidentiated.

The transplantability of the cultured corneal epithelium is previously tested by grafting onto nude mice. The "take" is always good and a 4-5 layer stratified squamous epithelium developed. When epithelial sheets bearing goblet cells are grafted, the cells are maintained also *in vivo.*

### Patient 1

A 62 years old caucasian male suffering from extensive right eye scarring was sent for consultation. He suffered from an alkali burm in his right eye 22 years before. He underwent penetrating keratoplasty two years afterwards; the graft rapidly failed and perforated with loss of the lens. Slit-lamp examination showed extensive limbal/corneal scarring covering the entire limbal/corneal surface, pannus and comeal neo-vascularization. There were extensive irido/corneal synechiae; no other details of ocular structures were visible. Visual acuity was reduced to hand movements with good projections in the 4 quadrants; ultrasonography showed a normal posterior pole. He was not considered a candidate for a repeated penetrating keratoplasty due to the poor conditions of his epithelium, and he was offered the option of an autologous limbal/corneal epithelium transplantation as a preparation for a future keratoplasty. Cells obtained from his left healthy limbus were cultured; when the autograft was ready, the right eye was prepped and draped and topical anesthesia with a lid block using 2% plain xylocaine were given. The cornea and the limbus were debrided with a blunt knife and scissors, and 360° perytomy extended for 2 mm beyond the limbus was performed. The cultured epithelial graft was cut to a suitable diameter and placed on the prepared host bed. After graft placement, the petrolatum gauze was gently removed under a microscope. A soft therapeutic hydrophilic contact lens was then placed and the eye was patched tightly for three days. Topical prednisolone acetate and chloramphenicol were administered B.I.D. for ten days, followed by artificial tears Q.I.D. and fluorometholone 0.3% B.I.D. The contact lens was removed after 2 weeks; at that time, the cornea was covered by a transparent, normal looking epithelium. At one month, slit-lamp examination showed no signs of intraocular inflammation. Sub-epithelial and stromal corneal scarring were still present. Fluorescein revealed minimal punctate staining of the corneal epithelium. Visual acuity was still hand movements. Four months later, the patient underwent a penetrating keratoplasty combined with coreplasty and anterior chamber intraocular lens implantation. Six months after the keratoplasty the grafted cornea was covered by a transparent epithelium and neo-vascularization was absent. The patient recovered a best corrected visual acuity of 0.7. Only at the last follow-up (16 months after penetrating keratoplasty), the patient give his permission for a corneal biopsy.

A 1 mm² biopsy was taken from the cornea (2.0 mm frcm the limbus), stained with ematoxylin-eosin and with the AE5 moAb, and subjected to PAS reaction as described under Methods. Histology showed a four to five layer stratified squamous epithelium with a cuboid basal layer, resembling a normal corneal epithelium. The epithelium showed no papillary structures, lyed on a avascular stroma with a well defined Bowman's layer, was devoided of goblet cells as well as of other cell types and showed an uniform PAS staining. The specimen was then stained with the moAb (AE5) against the corneal specific K3 keratin. A uniform positive staining was present. Conjunctival epithelium obtained from an age matched cadaver was negative. The sum of these data indicates that the epithelium obtained *in vitro* from limbal cells, regenerated a normal corneal epithelium on the patient.

### Patient 2

A 30 years old caucasian male with recurrent pterygium in his left eye was sent for consultation. Elsewhere, he underwent twice simple pterygium removal, followed by a third removal associated with β-radiation. A further recurrence was treated by an autograft of bulbar conjunctiva from his right eye. One month later a further pterygium started to form and was noticed to be aggressively growing; the patient was referred to us for further management. The right eye was within normal limits. The left eye had a pterygium extending horizontally for 3.5 mm over the cornea, which was scarred to mid-stroma, presumably from the previous surgical procedures. The pterygium itself was heavily vascularized; the nasal bulbar conjunctiva was injected and scarred. Globe movements were unrestricted. Since recurrent pterygium can be due to localized limbal zone deficiency or alteration, it was proposed to the patient to graft his eye with autologous epithelium cultured from the fellow limbus. When the graft was ready the recipient bed was prepared by removing the pterygium with a superficial keratectomy: the corneal and bulbar epithelium were also removed for 120° in the nasal sector, extending the dissection for 5 mm beyond the limbus. The cultured epithelial graft was cut in a semilunar shape, and placed as to cover the nasal side of cornea, limbus and sclera. A soft hydrophillc contact lens was positioned and the eye was pressure-patched for 3 days. Topical prednisolone acetate and tobramycin B.I.D. were administered, and the contact lens was removed aftar 3 weeks. At that time, the corneal and the conjunctival epithelium had completely healed. At the last follow-up, 19 months after surgery, there was no sign of pterygium recurrence. The eye was quiet and confortable and no treatment was needed. Slit lamp examination showed the pre-existing stromal scarring of the cornea. The patient did not consent to a biopsy for histological examination.

### Patient 3

A 65 years old caucasian male, affected by inactive bilateral ocular cicatricial pemphigoid, showed in both eyes symblepharon, corneal scarring, neovascularization and pannus of the ocular surface, more severe in his right eye. The patient was complaining of burning and tearing and needed frequent application of ocular lubricants. Visual acuity was reduced in both eyes to hand movements with good projections in the 4 quadrants. Cells were cultured from a 1 mm² biopsy taken from a small healthy looking area of his left limbus. The corneal epithelium and the pannus were removed on the left eye with a blunt knife and scissors and 360° perytomy extended for 2 mm beyond the limbus was performed. Graft application and post-operative treatment were as in patient 1. At one month, slit lamp examination showed no intraocular inflammation. Sub-epithelial corneal scarring and neo-vascularization were still present. Fluorescein drops revealed minimal punctate staining of the corneal epithelium. Visual acuity had improved to 0.1. At the last follow-up, seven months later, the cornea was covered with transparent, normal looking epithelium, fluorescein drops revealed punctate staining, and the visual acuity was 0.05. Neo-vascularization was greatly reduced. During this period the patient manifested great satisfaction for the improvement in his vision, a substantial decrease of burning and tearing and did not need application of ocular lubricants. The patient did not give his permission for an histological examination.

### EXAMPLE 3 Corneal tissue fitted to a semisolid carrier

The ocular epithelium obtained as described in the Example 1, both under fresh and frozen conditions, is placed on a Petri dish with its external surface upwards. The convex surface of a semirigid lens out of Lidofilcon A with 70 % of water, diameter 14.3 mm, thickness at the middle 140 µm, and curvature ratio 8.7 mm (Plano T from Baush & Lomb), is placed on the mucose external surface, and then, the borders of the mucose are turned onto the concave lens surface. The convex surface of a second lens, diameter of which is the same of the first lens, is placed so that it mated the concave surface of the first lens in order to maintain the mucose borders in its correct position.

Then the mucose-lens assembly is turned so that the convex surfaces became concave surfaces, and "viceversa", so that the epithelium basis layer, which shall be in contact with the eye surface to be transplanted, covers the concave surface of the first lens.

## Claims

1. A process to grow *in vitro* differentiated ocular surface epithelial cells, comprising the following steps:
- drawing biopsies from a donor site of the ocular surface of the eye;
- treating said biopsies with cell dissociating agents to obtain dissociated cells;
- cultivating *in vitro* said cells on a solid carrier until a confluent layer of differentiated cells having at least one phenotypic feature of cells of the donor site is obtained;
- separating said layer from said solid carrier by means of a detaching agent.

2. A process to grow *in vitro* differentiated ocular surface epithelial cells according to the claim 1 wherein said donor site comprises eye limbus and/or perilimbus areas.

3. A process to grow *in vitro* differentiated ocular surface epithelial cells according to the claim 1 wherein said donor site comprises the eye fornix and/or conjunctiva.

4. A process to grow *in vitro* differentiated ocular surface epithelial cells according to any of previous claims wherein said biopsies are freshly used.

5. A process to grow *in vitro* differentiated ocular surface epithelial cells according to any of claims from 1 to 3 wherein said biopsies are frozen before using.

6. A process to grow *in vitro* differentiated ocular surface epithelial cells according to any of previous claims wherein said treatment with cell dissociating agents comprises more than one incubation therewith, each one followed by removal of the supernatant containing dissociated cells.

7. A process to grow *in vitro* differentiated ocular surface epithelial cells according to any of previous claims wherein said dissociating agents comprise trypsin and bivalent ion chelating agents, more preferably EDTA, and most preferably said trypsin has a concentration in the range 0.01-0.1 % (w/v), and said EDTA has a concentration in the range 0.005-0.02 % (w/v).

8. A process to grow *in vitro* differentiated ocular surface epithelial cells according to any of previous claims wherein said *in vitro* culture is carried out in the presence of feeder layer of lethally irradiated cells and with a suitable culture medium for epithelial cells, preferably added with general or specific epithelium growth factors.

9. A process to grow *in vitro* differentiated ocular surface epithelial cells according to any of previous claims wherein said culture comprises several cell passages in order to obtain no primary cultures, preferably two steps in order to obtain secondary cultures.

10. A process to grow *in vitro* differentiated ocular surface epithelial cells according to any of previous claims wherein said cultures are frozen.

11. In *vitro* culture of a differentiated ocular surface epithelial cells from an ocular biopsy expressing at least one phenotypic feature of cells of the donor site.

12. *In vitro* culture of a differentiated ocular surface epithelial cells obtained according to the process of any of previous claims.

13. Ocular epithelium-carrier complex to be used for eye transplantation, comprising a solid carrier and a culture of differentiated ocular surface epithelial cells according to claim 11 or 12.

14. Ocular epithelium-carrier complex according to claim 13 wherein said solid carrier comprises a sterile gauze.

15. Ocular epithelium-carrier complex according to claim 13 wherein said solid carrier comprises semirigid lens.

16. Process for the preparation of an ocular epithelium-carrier complex to be used for eye transplantation, including semirigid lens and a culture of differentiated ocular surface epithelial cells, comprising the steps of:
- positioning said culture with the outer surface thereof upwards and leaning the convex surface of a first semirigid lens against it;
- turning the borders of said culture onto the concave surface of said first lens and fastening said borders by positioning the convex surface of a second lens on the concave surface of said first lens;
- turning the ocular epithelium-carrier complex in such a way, that the convex surfaces of said lens become concave and "viceversa", so that the epithelium basal layer, which is opposite to said outer surface, covers the concave surface of the first lens.
